# FASCICULE DE BREVET EUROPEEN

(11) **EP 2 870 260 B1**
(45) Date de publication et mention de la délivrance du brevet: **30.08.2017**
(21) Numéro de dépôt: 13734714.2
(22) Date de dépôt: 01.07.2013
(51) Int. Cl.: B01L 3/00, B01L 7/00, G01N 21/64, C12Q 1/68

(54) **PROCEDE ET DISPOSITIF POUR LA DETECTION RAPIDE DE SEQUENCES NUCLEOTIDIQUES AMPLIFIEES**
VERFAHREN UND VORRICHTUNG ZUM SCHNELLEN NACHWEIS VON AMPLIFIZIERTEN NUKLEOTIDSEQUENZEN
METHOD AND DEVICE FOR RAPID DETECTION OF AMPLIFIED NUCLEOTIDE SEQUENCES

(30) Priorité: 04.07.2012 BE 201200458
(43) Date de publication de la demande: 13.05.2015
(73) Titulaire: Coris Bioconcept SPRL, 5032 Gembloux (BE)
(72) Inventeur: LECLIPTEUX, Thierry, B-5100 Wépion (BE); MERTENS, Pascal, B-5340 Haltinne (BE); AVRAIN, Laetitia, B-1040 Etterbeek (BE); OTE, Isabelle, B-4000 Liège (BE); SMEKENS, Valérie, B-5000 Namur (BE)
(74) Mandataire: Pronovem
(86) Numéro de dépôt international: PCT/EP2013/063777
(87) Numéro de publication internationale: WO 2014/005969

(56) Documents cités:
- WO-A1-2004/099438
- WO-A2-2004/065010
- WO-A2-2009/080817
- US-A1- 2003 073 121
- KOPP M U ET AL: "CHEMICAL AMPLIFICATION: CONTINUOUS-FLOW PCR ON A CHIP", SCIENCE, AMERICAN ASSOCIATION FOR THE ADVANCEMENT OF SCIENCE, WASHINGTON, DC; US, vol. 280, 1 mai 1998 (1998-05-01), pages 1046-1048, XP002930052, ISSN: 0036-8075, DOI: 10.1126/SCIENCE.280.5366.1046
- CREWS N ET AL: "Continuous-flow thermal gradient PCR", BIOMEDICAL MICRODEVICES, KLUWER, DORDRECHT, NL, vol. 10, 1 avril 2008 (2008-04-01), pages 187-195, XP002571191, ISSN: 1387-2176, DOI: 10.1007/S10544-007-9124-9 [extrait le 2007-09-15]
- DAFENG CHEN ET AL: "An integrated, self-contained microfluidic cassette for isolation, amplification, and detection of nucleic acids", BIOMEDICAL MICRODEVICES, KLUWER ACADEMIC PUBLISHERS, BO, vol. 12, no. 4, 17 avril 2010 (2010-04-17) , pages 705-719, XP019814145, ISSN: 1572-8781
- WANG JING ET AL: "A disposable microfluidic cassette for DNA amplification and detection", LAB ON A CHIP, ROYAL SOCIETY OF CHEMISTRY, vol. 6, no. 1, 1 janvier 2006 (2006-01-01) , pages 46-53, XP002461321, ISSN: 1473-0197, DOI: 10.1039/B511494B
- Z. CHEN ET AL: "A Microfluidic System for Saliva-Based Detection of Infectious Diseases", ANNALS OF THE NEW YORK ACADEMY OF SCIENCES, vol. 1098, no. 1, 1 janvier 2007 (2007-01-01), pages 429-436, XP055052232, ISSN: 0077-8923, DOI: 10.1196/annals.1384.024

## Description

### Objet de l'invention

La présente invention est relative à un procédé et un dispositif pour la détection rapide de séquences génétiques (amplicons) provenant de l'amplification (génétique) (enzymatique) d'une séquence génétique originale et spécifique présente dans un échantillon biologique.

Le procédé est de préférence conçu pour des applications diagnostiques sur des échantillons comprenant de multiples séquences génétiques (oligonucléotidiques) cibles différentes, (i) pour une détection rapide et efficace de séquences nucléiques spécifiques dans un échantillon, et une corrélation de cette détection à différentes pathologies et maladies d'un individu dont est issu l'échantillon testé, en particulier des maladies infectieuses, telles que des bactériémies, les maladies infectieuses respiratoires ou entériques, y compris les formes résistantes à des agents thérapeutiques tels que des antibiotiques, (ii) pour identifier des contaminations de produits alimentaires, ou (iii) pour des tests pronostiques, notamment de la présence éventuelle de marqueurs cancéreux dans des échantillons.

### Etat de la technique

Parmi les procédés d'amplification (génétique), la PCR (Polymerase Chain Reaction) est la technique la plus utilisée en biologie moléculaire pour obtenir l'amplification spécifique d'une seule ou de plusieurs copie(s) d'une séquence nucléotidique originale et spécifique éventuellement présente dans un échantillon biologique. Cette technique, par cycles successifs d'amplification (génétique), permet de générer des millions, voire des milliards de copies de la séquence originale et spécifique. La méthode d'amplification par PCR comporte des cycles thermiques répétés de chauffage et de refroidissement à plusieurs (deux ou trois) températures spécifiques pour la dénaturation des séquences nucléotidiques, l'hybridation des amorces et l'élongation enzymatique des séquences nucléotidiques au départ des amorces hybridées.

Cette méthode est réalisée grâce à un instrument programmable qui chauffe et refroidit une chambre de réaction stationnaire contenant les séquences génétiques cibles de l'échantillon à amplifier par des cycles thermiques successifs, en particulier des cycles répétés et successifs de hausses et de baisses de température.

La vitesse de l'amplification est limitée par la vitesse de chauffe et de refroidissement des blocs chauffants de l'instrument.

Afin de diminuer le temps total des amplifications (multiples) de la PCR, différentes approches ont été proposées. L'une d'entre elles consiste à réaliser une amplification PCR sous flux dynamique continu.

Cette technique permet un passage de l'échantillon sous flux constant au travers d'un (micro)canal qui passe par deux ou trois zones spatiales différentes, chacune ayant une température constante.

Un tel dispositif est notamment décrit dans l'article de Kopp et al (1998, Science 280, 1046-1048). Ce dispositif comporte un canal passant à plusieurs reprises au travers de trois zones de températures différentes pour la dénaturation, l'hybridation et l'élongation. L'échantillon est introduit à une des extrémités du canal et pompé dans une direction vers l'autre extrémité du canal où l'échantillon amplifié est récupéré pour être analysé.

L'amplification (génétique) sous format miniaturisé et en flux dynamique continu (constant) nécessite également une détection des séquences nucléotidiques amplifiées.

Dans l'article de Kopp et al, les échantillons amplifiés sont analysés par électrophorèse sur gel coloré au bromure d'éthidium à l'extérieur du dispositif d'amplification.

La PCR étant une technique d'amplification extrêmement sensible, de très faibles quantités de séquences nucléotidiques (notamment provenant d'amplifications précédentes) aptes à contaminer l'échantillon peuvent être aussi amplifiées, engendrant des résultats faux positifs. En vue d'éliminer cet inconvénient, l'approche la plus efficace consiste à intégrer les étapes d'amplification (par PCR) et les étapes de la détection des produits amplifiés dans un même dispositif fermé et jetable (à usage unique).

De tels dispositifs intégrés existent pour une détection, par exemple par électrophorèse capillaire ou par hybridation sur micro-damier. Cependant, de tels procédés et dispositifs de mesure et d'analyse sont généralement complexes et coûteux. De plus, la détection sur ces dispositifs peut être perturbée par l'émission de microbulles générées lors de l'étape d'amplification (génétique) préalable.

Par conséquent, il existe un besoin de simplification de ces procédés et dispositifs afin d'en simplifier l'usage et l'efficacité, d'en réduire le coût et d'en augmenter la rapidité d'utilisation.

### Buts de l'invention

La présente invention vise à fournir un procédé et un dispositif d'amplification (génétique), de préférence par PCR en flux dynamique continu, combinés à un procédé et un dispositif de détection qui soient simples et peu coûteux et qui ne présentent pas les inconvénients de l'état de la technique.

Un but particulier de la présente invention est de fournir un tel procédé et dispositif qui permettent une détection rapide, mais également efficace, afin d'obtenir un résultat d'analyse de la présence de séquences nucléiques spécifiques dans un échantillon, de préférence un diagnostic rapide et non faussé de l'échantillon, et éventuellement une corrélation de cette détection à différentes pathologies et maladies de l'individu dont est issu l'échantillon, en particulier, des maladies infectieuses, telles que les bactériémies, les maladies infectieuses respiratoires ou entériques y compris les formes résistantes à des agents thérapeutiques, en particulier des antibiotiques.

### Résumé de l'invention

Un premier aspect de la présente invention est relatif à une méthode d'amplification, de préférence par PCR, et de détection d'au moins une, et/ou jusqu'à plusieurs dizaines (détection multiplexe), séquence(s) nucléotidique(s) cible(s) éventuellement présente(s) dans un échantillon biologique, de préférence (i) un échantillon extrait d'un animal ou d'un individu tel qu'un mammifère, plus particulièrement d'un humain, ou (ii) une contamination d'une composition alimentaire, ladite méthode comprenant les étapes consécutives suivantes :
a) fournir un dispositif comprenant :
   - un canal ayant une section comprise entre environ 0,01 mm et environ 10 mm,
   - une tigette en communication fluidique avec ledit canal, ladite tigette comprenant:
   - soit des premières (une ou plusieurs) sonde(s) complémentaire(s) et spécifique(s) d'au moins une première partie de séquence de la ou des dite(s) séquence(s) nucléotidique(s) cible(s) amplifiée(s) ou
   - soit un réactif de capture constitué d'une première molécule d'une paire de molécules différentes et complémentaires, et apte à fixer spécifiquement une deuxième molécule (différente) de ladite paire (de molécules différentes et complémentaires), cette seconde molécule étant fixée (directement via une amorce s'incorporant dans l'amplicon pendant l'amplification, de préférence la PCR, ou indirectement via une sonde complémentaire) à ladite première partie de séquence de la ou des dite(s) séquence(s) nucléotidique(s) cible(s) amplifiée(s), et de préférence
   - des moyens pour générer au moins quatre (trois) zones de températures, de préférence différentes et constantes, trois (au moins deux) desdites zones étant situées à des emplacements différents du canal et la quatrième (troisième) zone étant située au niveau de la tigette.
Ces moyens pour générer les trois (au moins deux) zones de températures, de préférence différentes et constantes, sont situés à des emplacements différents du canal apte à générer des étapes d'amplification (génétique), tandis que la quatrième (troisième) zone située au niveau de la tigette est maintenue constante à une valeur inférieure aux trois (au moins deux) autres zones, pour un traitement efficace de l'hybridation sur la membrane (tigette) en flux capillaire (chromatographie capillaire).

Dans le procédé de l'invention sus-décrit, on introduit ensuite (selon une étape b)) dans le canal du dispositif, en particulier du dispositif de l'invention, une solution comprenant l'échantillon ainsi que des réactifs (composés et éventuellement des additifs) pour l'amplification (génétique), de préférence par PCR, et éventuellement une RT-PCR ou une MLPA (Multiplex Ligation-dependent Probe Amplification), de la séquence nucléotidique cible. On génère ainsi (selon l'étape c)) des séquences amplifiées, et éventuellement marquées, de la ou des séquences nucléotidique(s) cible(s) en faisant circuler la solution selon un flux (dans un fluide) dynamique, de préférence constant (ou continu) dans ledit canal avant de mettre en contact (selon l'étape d)) lesdites séquences amplifiées avec la tigette, et ainsi générer (selon l'étape e)), par hybridation entre les premières sondes et lesdites séquences amplifiées (et éventuellement des secondes sondes marquées, ou des marqueurs intercalant de l'ADN), un complexe formant un signal de détection mesurable.

Dans la méthode de l'invention, les étapes b) à e) sont générées dans le dispositif de l'étape a) conçu de manière fermée et donc avantageusement non (ou peu) contaminable par d'autres séquences génétiques.

Selon la méthode de l'invention, les séquences amplifiées sont générées dans le canal comprenant de multiples boucles passant sur les deux (ou trois) zones de températures différentes, constantes et configurées pour les cycles multiples d'amplification (génétique), de préférence par PCR.

Par conséquent, dans le canal, les multiples boucles passent sur au moins deux (de préférence deux ou trois) zones de températures différentes, constantes et configurées pour les cycles multiples d'amplification (génétique), de préférence par PCR.

De préférence, dans la méthode de l'invention, la solution est introduite (et poussée) dans le canal par une pompe-seringue permettant un déplacement de l'échantillon dans le canal selon un flux dynamique constant. La solution peut également être introduite et déplacée en flux dynamique constant dans ledit canal par un dispositif d'aspiration. Un flux dynamique constant en circuit fermé peut également être appliqué à l'aide d'une pompe, telle qu'une pompe péristaltique (ou pompe à galets).

Dans la méthode de l'invention, pour faciliter la détection, les réactifs pour l'amplification (génétique), de préférence par PCR, sont notamment des paires d'amorces d'amplification (génétique) qui peuvent être éventuellement marquées. Selon une alternative à cette méthode, on peut aussi obtenir un marquage de la séquence amplifiée (amplicon) par un intercalant de l'ADN marqué (fluorescent par exemple).

De préférence, au moins une des amorces de chaque paire est marquée.

De préférence, le marqueur de l'amorce ou de la séquence amplifiée (amplicon) est choisi parmi le groupe constitué par les particules métalliques, en particulier les particules d'or, les particules colloïdales, les particules de polystyrène, les particules colorées, les particules (para)-magnétiques ou les éléments fluorescents.

Selon une forme d'exécution préférée de la méthode de l'invention, le marqueur est un marqueur fluorescent, tel que la cyanine (Cy5) ou un marqueur ayant des propriétés fluorescentes similaires.

De manière avantageuse dans la méthode de l'invention, le signal de détection est constitué d'une ou plusieurs lignes formées par le marqueur, ou un ou plusieurs points formés par le marqueur, de préférence le signal est constitué (d'un réseau) de 4, 6, 8, 9, 10, 12, 14 ou 16 points, voire 32 points ou plus, formés par le marqueur et donc présents sur la surface de la tigette. Le choix du nombre de points formés par le marqueur sur la surface de la tigette est sélectionné en fonction de la résolution de détection et de la facilité à obtenir une photo (image) de l'ensemble des points et une discrimination entre les différents points.

La méthode de l'invention peut également mettre en contact une partie de la séquence des séquences nucléotidiques amplifiées, non hybridée avec les premières sondes, avec une ou plusieurs secondes sondes marquées de manière à favoriser une détection de type sandwich.

La méthode de l'invention permet une amplification et une détection de tout type de séquence nucléotidique cible qu'elle soit simple brin, double brin, ou partiellement double brin, ou une séquence d'ADNc rétro-transcrite à partir d'une séquence d'ARN.

Selon une forme d'exécution préférée de l'invention, la méthode comporte une étape de MLPA (Multiplex Ligation-dependent Probe Amplification) ou une étape de RT-PCR.

Dans la méthode de l'invention, la lecture du signal est également de préférence corrélée à l'identification d'une ou plusieurs infection(s), ou contamination d'une composition alimentaire par un ou plusieurs agent(s) éventuellement pathogène(s), tel que des plantes génétiquement modifiées, des bactéries ou des virus, et/ou à l'identification de la résistance d'un ou plusieurs agent(s) éventuellement pathogène(s) à un ou plusieurs traitement(s) thérapeutique(s), en particulier une résistance à l'action d'un ou plusieurs antibiotique(s) lorsque l'agent éventuellement pathogène est une bactérie.

L'avantage de la méthode de l'invention est sa rapidité, car elle peut être exécutée rapidement, en moins de 90 minutes, de préférence en moins de 60 minutes, voire dans des conditions préférées en moins de 30 minutes.

La méthode de l'invention permet également une amplification et une détection de séquences nucléotidiques cibles qui peuvent être multiples et différentes, et présentes simultanément dans le même échantillon (conditions multiplexes). Le nombre de ces séquences nucléotidiques peut être de 5, 10, 15, 20, 25, 50, 100, 200, 400, 600, 800, 1000 ou plus (de séquences nucléotidiques cibles différentes).

La présente invention est également relative à un dispositif d'amplification et de détection (rapide) d'au moins une séquence nucléotidique cible, comprenant les moyens pour la mise en oeuvre de la méthode de l'invention, et comprenant au moins un canal ayant un section comprise entre environ 0,01 mm et 10 mm, une tigette en communication fluidique avec ledit canal, ladite tigette comprenant soit des premières sondes complémentaires et spécifiques d'une première partie de séquence des séquences nucléotidiques cibles amplifiées (à détecter) ou soit un réactif de capture constitué d'au moins une première molécule d'une paire de molécules différentes et complémentaires (telles que la streptavidine/biotine, l'avidine/biotine ou la polystrepatividine/biotine), et apte à fixer la seconde molécule de ladite paire, ladite seconde molécule étant fixée (directement via une amorce s'incorporant dans l'amplicon pendant l'amplification, de préférence la PCR, ou indirectement via une sonde complémentaire) à la première partie de séquence des séquences nucléotidiques cibles amplifiées, de préférence ledit canal et ladite tigette étant présents dans deux chambres différentes.

De manière avantageuse, le dispositif peut comprendre également des moyens pour générer au moins quatre (trois) zones de températures différentes et constantes, trois (au moins deux) zones étant situées à des emplacements différents du canal et la quatrième (troisième) zone étant située au niveau de la tigette; ledit canal pouvant comporter de multiples boucles passant sur les trois (deux) zones de températures différentes, constantes et configurées pour les cycles multiples d'amplification (génétique), de préférence par PCR. Le dispositif peut comprendre également à l'extrémité du canal, une pompe-seringue assurant l'introduction et le flux dynamique de préférence constant (ou continu) de l'échantillon au sein du canal, ou comprendre d'autres moyens pour assurer le flux dynamique de préférence constant (continu) de l'échantillon au sein du canal par aspiration, ou notamment une pompe péristaltique (pompe à galets), ainsi que des moyens de traitement préalable à l'échantillon par MLPA ou RT-PCR ou d'autres traitements préalables de l'échantillon.

Dans le dispositif de l'invention, la tigette comporte des premières sondes (et éventuellement des secondes sondes (éventuellement marquées)) aptes à générer un signal de détection sous forme de lignes ou de points sur la surface de la tigette après hybridation des séquences nucléotidiques amplifiées. Ce signal de détection peut être constitué (d'un réseau) de 4, 6, 8, 9, 10, 12, 14 ou 16 points, voire 32 points ou plus.

Un autre aspect de l'invention concerne une trousse d'amplification, de préférence par PCR, et de détection, comprenant les moyens du dispositif de l'invention sus-décrit, ainsi que les milieux (réactifs) d'une amplification (génétique), de préférence par PCR, et d'une détection chromatographique sur tigette. Ces moyens sont de préférence des sondes, de préférence marquées, et/ou éventuellement des moyens de dilution d'échantillons, en particulier une solution tamponnée, ainsi que des éléments habituellement utilisés pour une amplification (génétique), de préférence par PCR, en particulier des paires d'amorces (uniques ou universelles), dont au moins une amorce de chaque paire est éventuellement marquée, ainsi qu'une ou plusieurs séquence(s) de contrôle interne de l'efficacité d'amplification (afin de vérifier si un résultat négatif est un vrai négatif).

De préférence, le marqueur étant choisi parmi le groupe constitué par les particules métalliques, en particulier les particules d'or, les particules colloïdales les particules colorées, les particules de polystyrène, les particules para(magnétiques) ou les éléments fluorescents, de préférence dans la trousse de l'invention, le marqueur est un élément fluorescent, plus particulièrement la cyanine (Cy5) ou un marqueur ayant des propriétés fluorescentes similaires.

La présente invention sera décrite plus en détail dans la forme d'exécution préférée de l'invention en référence aux figures annexées.

### Brève description des figures

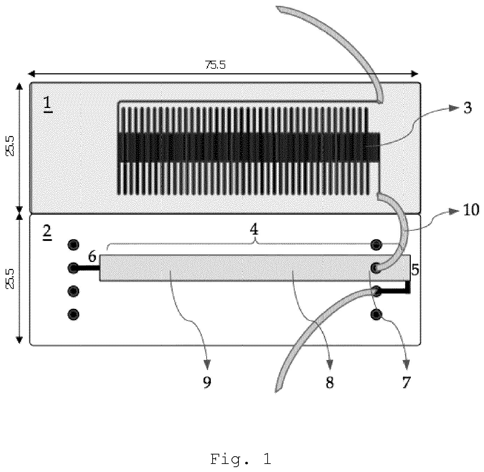

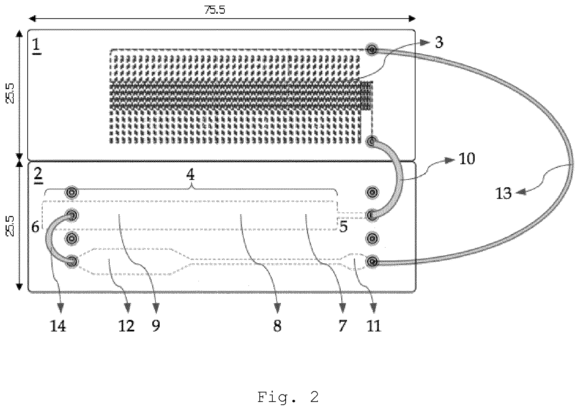

Les figures 1 et 2 représentent de manière schématique les caractéristiques du dispositif de l'invention selon un premier mode de réalisation. Le dispositif comprend une première partie comprenant des moyens destinés à une amplification (génétique) de séquences d'acides nucléiques en flux dynamique constant, combinée à une seconde partie comprenant des moyens permettant la détection des séquences amplifiées sur membrane (tigette) par une technique d'oligochromatographie. Les deux parties sont reliées par des moyens de transfert des séquences amplifiées, tel qu'un tuyau (tube) de communication fluidique.
La figure 3 représente de manière schématique les caractéristiques du dispositif de l'invention selon un deuxième mode de réalisation dans lequel les deux parties sont reliées par un canal intégré dans la structure du dispositif.
La figure 4 représente de manière schématique les différentes façons de disposer les moyens de détection sur la tigette afin de générer par hybridation (selon l'étape e)) un complexe formant un signal de détection mesurable.

### Description détaillée de l'invention

Le dispositif tel que représenté à la figure 1 comprend des moyens de traitement microfluidique d'un échantillon permettant une amplification (génétique) rapide et spécifique de séquences (d'acides nucléiques), en particulier une amplification de type PCR en flux dynamique constant (ou continu), combinés à des moyens de détection de ces séquences nucléotidiques amplifiées (amplicons).

Tel que représenté à la figure 1, le dispositif de l'invention comprend une première zone ou chambre 1 d'amplification (génétique) par PCR comprenant les différents moyens adéquats pour effectuer une telle amplification (génétique), et une deuxième zone ou chambre 2 de détection comprenant les différents moyens adéquats pour effectuer une telle détection des séquences génétiques amplifiées provenant de la chambre 1.

Un dispositif tel que décrit dans la chambre 1 est déjà connu de l'état de la technique et peut comporter différents moyens pour une amplification (génétique) de préférence par PCR, en microfluidique et de préférence sous flux dynamique constant.

Ces moyens comprennent un canal 3 réalisé en verre, en quartz ou en plastique, en particulier en polycarbonate (Bayer Material Science) ou en copolymères de cyclo-oléfines, et fabriqué par moulage par injection. Les caractéristiques de microstructure de ce canal sont de préférence réalisées par un traitement classique de photolithographie.

L'ouverture du canal 3 est éventuellement adaptée dans la première partie du dispositif pour faciliter l'introduction et le traitement préalable de l'échantillon à tester et éventuellement dilué. Un tel échantillon, de préférence une solution comprenant l'échantillon, peut être intégrée dans le canal 3 par différents moyens connus de l'homme de l'art, notamment des micro-seringues.

Le canal 3 a de préférence une configuration en serpentin dont les dimensions peuvent varier, mais qui comprend un certain nombre de boucles aptes à réaliser les différentes étapes nécessaires d'amplification (génétique), et est mis en contact avec des moyens de chauffe thermique précise de zones de ces boucles. Ces moyens peuvent consister en des dispositifs Peltier ou des résistances chauffantes ou des blocs chauffants en aluminium et des capteurs adéquats.

Le canal 3 a de préférence une longueur comprise entre environ 1500 mm et environ 2000 mm, voire plus jusqu'à 3000 mm ou plus, généralement de l'ordre de 1850 mm, une profondeur d'environ 100 µm et une largeur (diamètre) d'environ 200 µm. Le motif en serpentin avec de multiples boucles est prévu de manière à pouvoir faire un certain nombre de cycles d'amplification (génétique), de préférence plus de 15, 20, 25 ou 30 voire 50 cycles d'amplification (génétique), de préférence plus de 35 cycles d'amplification (génétique), plus particulièrement plus de 40 cycles d'amplification (génétique), de préférence 41 cycles d'amplification (génétique) dans le présent exemple.

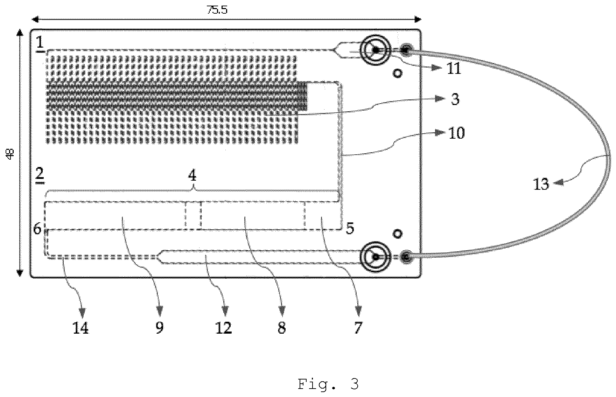

Ces cycles d'amplification (génétique) peuvent également être précédés d'une étape de pré-dénaturation et suivis par une étape d'élongation finale (post-élongation), et le dispositif de l'invention peut également comporter un ou plusieurs réservoir(s) 11 et 12 permettant de contenir l'échantillon à analyser et le ou les tampon(s) utile(s) à des étapes du procédé de l'invention, comme le tampon d'oligochromatographie ou de lavage destiné à éliminer les liaisons (hybridations) non spécifiques des séquences amplifiées sur le support de détection. Ces réservoirs sont de préférence remplis par des ouvertures de type « luer », fermées par des bouchons. Avantageusement, le dispositif comporte également un tuyau 13, de préférence flexible et formant une boucle et apte à rentrer en contact avec une tête de pompe, c'est-à-dire avec les éléments (galets) d'une pompe péristaltique (pompe à galets). Ce tuyau 13 relie, via des connecteurs de type « olive », le réservoir 11 destiné à contenir l'échantillon et le réservoir 12 contenant le tampon pour assurer un déplacement fluidique (flux dynamique constant) de l'échantillon et du tampon, par la mise en oeuvre par la dite pompe, au travers du dispositif de l'invention. Un autre moyen de liaison (tuyau) 14 permet aussi de relier les moyens de détection présents dans la seconde zone ou chambre 2 (tigette 4) au réservoir 12. Ce système fermé tel que représenté à la figure 3 est donc (peu ou) non contaminable ni contaminant.

De préférence, le dispositif microfluidique est réalisé en une seule pièce et le canal 3 est mis en contact (via une communication fluidique 10, permettant le transfert des séquences amplifiées vers la seconde zone ou chambre 2) avec un moyen de détection présent dans la seconde zone ou chambre 2 du dispositif de l'invention. De préférence, la fin du canal 3 destiné à l'amplification (génétique) rejoint (est mis en contact avec) une tigette 4 permettant une détection des séquences génétiques amplifiées par un procédé dénommé oligochromatographie.

De préférence, les moyens (présents sur une tigette) destinés à une détection par oligochromatographie sont disposés dans une seconde zone ou chambre 2 séparée de la chambre 1 comprenant le canal 3. Les deux chambres sont en communication fluidique de sorte que le produit amplifié (la solution contenant les séquences amplifiées) peut être directement transféré dans la chambre 2 pour sa détection dans un système fermé et de préférence non contaminable ni contaminant.

Cette seconde chambre 2 comprend une tigette 4 incorporant trois zones ou régions. Ces trois zones sont constituées de membranes poreuses permettant une migration par capillarité d'un fluide et de ses composants (c'est-à-dire la solution comprenant les séquences amplifiées) d'une partie proximale 5 vers une partie distale 6. Ces trois zones ou régions sont une région d'application 7, une région de détection 8 et une région absorbante 9.

De manière préférée, la membrane de la zone d'application 7 est constituée de fibres de verre ou d'un autre matériau adéquat (polyester), la membrane de la zone de détection 8 est constituée de nitrocellulose, et la membrane de la région absorbante 9 est constituée de préférence de cellulose. D'autres types de structure et de matériaux peuvent être envisagés par l'homme de l'art pour améliorer les caractéristiques de migration par capillarité au sein de la tigette 4 ou pour améliorer l'intégration des éléments destinés à la détection sur leur support. L'homme du métier peut par exemple prévoir des structures de tigettes 4 dans lesquelles la zone d'application 7 est présente sur la même membrane que la zone de détection 8 ou sans zone ou région absorbante 9. On peut aussi envisager que la tigette 4 soit totalement recouverte de nitrocellulose et éventuellement dépourvue de zone absorbante.

Différentes zones ou régions peuvent comporter différents réactifs aptes à faciliter la détection des différentes séquences génétiques amplifiées mises en contact avec la tigette 4.

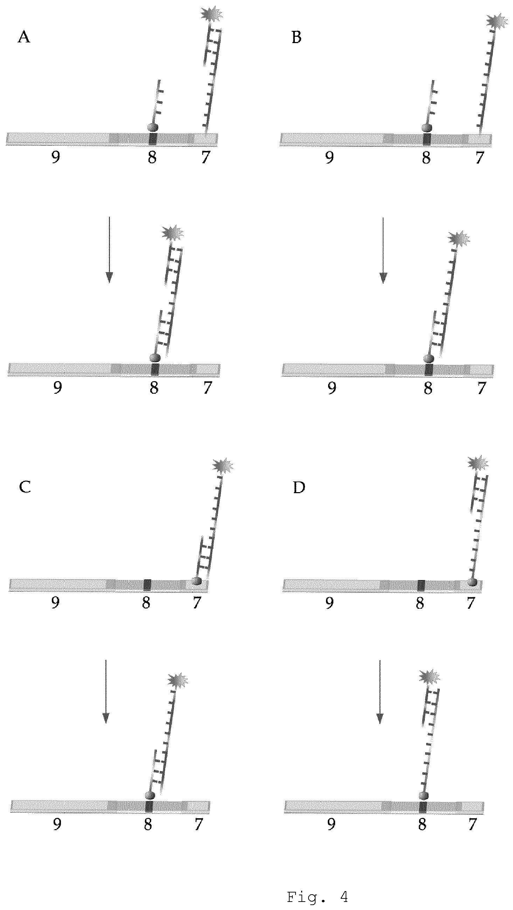

De préférence, les moyens de mise en contact du canal 3 d'amplification (génétique) se font sur la zone d'application 7 qui comprend les moyens d'hybridation spécifique des séquences génétiques amplifiées (Figure 4). De manière avantageuse et inattendue, cette application (via la communication fluidique 10) et le déplacement par capillarité sur la tigette 4 peut se faire directement par le flux de la solution comprenant les séquences amplifiées, sans nécessiter d'addition d'un quelconque solvant supplémentaire ou de sels spécifiques d'hybridation. De préférence, les volumes de solution emportés par le canal et déposés sur la tigette sont compris entre environ 10 µl et environ 50 µl, de préférence entre environ 30 µl et environ 10 µl, de préférence de l'ordre de 20 µl.

La représentation de la détection peut être une ou plusieurs ligne(s) de détection, ou un ou plusieurs point(s) de détection (ou tout autre figure, lettre, ligne, point ou chiffre) présent(e(s)) sur la membrane de la zone de détection 8, une ligne ou un point de détection étant spécifique d'une détection d'une ou de plusieurs séquence(s) nucléotidique(s) amplifiée(s) provenant de l'amplification réalisée dans le canal 3 d'amplification (génétique). De manière avantageuse, les réactifs de détection sont placés sur le support de la tigette selon un réseau afin de générer les figures adéquates facilement lisibles par un détecteur, de préférence un réseau de 4, 6, 8, 9, 10, 12, 14 ou 16 points, voire 32 points ou plus, aptes à comporter au moins un ou deux points de contrôle de la détection (au moins un point de contrôle d'amplification (hybridation spécifique de la séquence spécifique utilisée comme contrôle interne) et un contrôle de migration (détection du surplus des séquences amorces (éventuellement marquées) non utilisées lors de l'étape d'amplification), ainsi que des points de positionnement de la tigette).

Les moyens de détection présents sur la membrane, en particulier dans la zone de détection 8, sont constitués d'une première sonde de capture qui s'hybride (capture), dans des conditions spécifiques, à une partie de séquence spécifique d'une ou plusieurs séquence(s) nucléotidique(s) amplifiée(s) (Figure 4).

De préférence, cette première sonde de capture est constituée d'une séquence nucléotidique constituée d'acides nucléiques ou de molécules similaires (DNA, RNA, PNA, LNA, ANA, HNA, etc.). Un réactif porteur peut être lié à la première sonde de capture au travers d'un élément liant, tel qu'un haptène, un peptide ou toute autre molécule susceptible de fixer spécifiquement ledit réactif porteur.

De préférence, la première sonde de capture est couplée à une première molécule (premier élément) d'une paire (couple) de molécules différentes et complémentaires, telle que la biotine, elle-même fixée sur de la nitrocellulose via l'élément complémentaire du couple de molécules, telle que de l'avidine, de la streptavidine, de la polystreptavidine ou toute autre protéine liant la biotine, utilisé comme réactif de capture.

Au niveau de la détection, celle-ci est de préférence générée par l'excitation (l'émission) d'un marqueur fluorescent qui pourra provoquer (l'émission de) un ou plusieurs signaux fluorescents détectables sous forme de lignes ou de points, ou sous une autre forme géométrique (chiffre, dessin) sur la surface de la tigette 4. Ceux-ci pourront être visualisés via l'utilisation d'un détecteur de fluorescence. D'autres moyens seront choisis en fonction du type de marqueur utilisé (par exemple des moyens de mesure de la réflectance de sondes marquées à l'or).

Dans une première forme d'exécution, la détection se fera en deux étapes : une séquence nucléotidique (spécifique) cible amplifiée va s'hybrider avec une sonde spécifique complémentaire et marquée, présente dans la zone d'application 7 de la tigette 4, pour former un complexe entre la séquence cible et sa sonde spécifique marquée, complexe qui migrera sur la membrane vers la zone de détection 8 où il réagira avec la première sonde de capture fixée dans la zone de détection, formant ainsi un complexe de type sandwich. Dans ce complexe, une première partie de la séquence nucléotidique cible amplifiée est hybridée à une première sonde de capture permettant l'immobilisation de la séquence cible à un endroit précis de la tigette 4 et une deuxième partie de la séquence nucléotidique cible amplifiée est hybridée à une seconde sonde marquée permettant la détection du complexe formé et immobilisé sur la tigette 4 (Figure 4A). A ce moment-là, le complexe sera rendu visible et détectable par l'accumulation du marqueur.

Dans une deuxième forme d'exécution, la détection se fera en une étape : une séquence nucléotidique cible amplifiée et marquée (par exemple au moyen d'une amorce marquée s'incorporant dans l'amplicon pendant l'amplification, de préférence la PCR) va migrer sur la membrane de la tigette 4 vers la zone de détection 8, où la séquence cible marquée réagira avec la première sonde de capture fixée dans la zone de détection, formant ainsi un complexe. Dans ce complexe, une première partie de la séquence nucléotidique cible amplifiée est hybridée à une première sonde de capture permettant l'immobilisation de la séquence nucléotidique cible amplifiée à un endroit précis de la tigette 4 (Figure 4B). La présence d'un marqueur sur la séquence cible amplifiée permet la détection du complexe formé et immobilisé sur la tigette 4.

Après avoir fait migrer la séquence nucléotidique cible amplifiée et marquée vers la zone de détection 8, on peut éventuellement faire migrer une solution tamponnée visant à éliminer les amorces marquées en excès (qui n'ont pas été incorporées dans les séquences amplifiées) et qui pourraient éventuellement interférer avec la mesure du signal de détection. La solution tamponnée peut éventuellement être introduite (depuis un réservoir relié au dit canal 3) dans ledit canal 3 quelques secondes après avoir introduit l'échantillon dans ce même canal et suivre le flux du liquide subissant les étapes d'amplification et de détection du procédé.

De manière avantageuse, les moyens de détection du dispositif de l'invention ne sont nullement perturbés par l'introduction ou la formation de bulles dans le flux dynamique continu du canal, suite aux variations thermiques, comme cela pourrait être le cas en détection par électrophorèse capillaire ou sur micro-damiers.

Selon une forme d'exécution préférée de l'invention, la première sonde de capture est immobilisée sur la tigette 4 à des endroits précis préalablement à l'exécution du test de détection des séquences nucléotidiques cibles (Figure 4). Par exemple, un réactif de capture (première molécule d'un couple de molécules différentes et complémentaires) tel que de l'avidine, de la streptavidine ou de la polystreptavidine, peut être fixé sur la membrane dans toute la zone de détection 8, et la première sonde de capture, comportant la seconde molécule du couple de molécules différentes et complémentaires, tel que de la biotine, est ensuite déposée sur la membrane à des localisations précises.

La fixation est réalisée de préférence par dilution du réactif de capture et de la première sonde de capture dans un tampon adéquat, et par un dépôt de lignes ou de points sur la membrane, de préférence la membrane de nitrocellulose. Alternativement, la sonde est préalablement soit fixée sur une molécule porteuse (telle que une paire de molécules (d'éléments) complémentaires du type biotine-avidine, biotine-streptavidine, anticorps anti-biotine et biotine,...) ou sur un support non biologique telle qu'une microsphère blanche (non visible par le système de détection) avant dépôt, soit fixée (chimiquement) directement sur la membrane de nitrocellulose.

Dans le cas de dépôt de lignes, la vitesse de distribution du réactif de capture peut varier entre environ 50 mm et environ 10 mm par seconde, mais est de préférence fixée à une valeur d'environ 30 mm par seconde.

Le volume de matériel distribué peut varier entre environ 0,5 µl/cm et environ 3 µl/cm, mais de préférence de l'ordre d'environ 1 µl/cm.

Dans le cas de dépôt de points, le volume de matériel distribué peut varier entre 100 nl et 1 nl, mais est de préférence fixé à une valeur d'environ 10 nl.

La concentration des réactifs de capture peut varier entre environ 0,01 mg/ml (ou une concentration plus basse) ou environ 0,1 mg/ml et environ 5 mg/ml, de préférence de l'ordre de 1,5 mg/ml.

La concentration des premières sondes de capture peut varier entre environ 50 µM et environ 1 µM ou environ 1 nM ou une concentration plus basse, de préférence entre environ 30 µM et environ 5 µM, plus particulièrement de l'ordre de 10 µM.

Selon une forme d'exécution préférée de l'invention, le tampon utilisé pour la fixation consiste en une solution saline (NaCl) tamponnée avec un phosphate à un pH d'environ 7,2.

Lorsque le support est constitué d'une membrane de nitrocellulose, la tigette 4 est ensuite séchée pendant quelques secondes ou minutes (deux minutes ou plus) jusqu'à environ 72 heures à une température d'environ 50°C, ou une température d'environ 60°C ou plus.

Selon une forme d'exécution préférée de l'invention, la tigette 4 d'oligochromatographie utilisée dans le dispositif de l'invention a une taille d'environ 5 mm de largeur pour une longueur d'environ 57 mm, mais d'autres formes et formats de tigettes peuvent être utilisés.

Dans le dispositif et procédé de l'invention, les séquences amplifiées susceptibles d'être détectées peuvent être des séquences d'ADN, en particulier des séquences d'ADN simple brin ou double brin ou partiellement double brin, ainsi que des séquences d'ADNc obtenues par transcription inverse de séquences d'ARN.

L'étape de transcription inverse peut éventuellement être intégrée dans le procédé de l'invention au sein d'une unité séparée du dispositif de l'invention, par exemple dans une chambre ou un (micro)canal connecté(e) (éventuellement par des valves ou des pompes) au canal 3 où a lieu l'amplification (génétique), de préférence par PCR.

Le procédé et le dispositif de l'invention sont parfaitement adaptés pour l'amplification et la détection de multiples et différentes séquences nucléotidiques présentes dans le même échantillon (conditions multiplexes).

Le nombre de séquences nucléotidiques différentes pouvant être détectées dans le dispositif de l'invention peut être adapté selon les besoins nécessités par l'application. Le procédé et le dispositif de l'invention sont modulables et permettent d'adapter le nombre de sondes présentes sur la tigette selon le nombre de séquences nucléotidiques cibles à détecter.

Le nombre de paires d'amorces (spécifiques et/ou universelles) devra être adapté au nombre de séquences nucléotidiques cibles à amplifier.

Dans un mode de réalisation préféré, entre environ 2 séquences et environ 50 séquences nucléotidiques cibles, de préférence entre environ 5 séquences et environ 20 séquences nucléotidiques cibles sont amplifiées et détectées dans le même échantillon.

Au-delà d'un certain nombre de paires d'amorces spécifiques présentes dans la même solution d'amplification, l'efficacité de l'amplification (PCR) diminue.

Afin d'augmenter le nombre de séquences nucléotidiques cibles pouvant être détectées dans un même échantillon, il est possible d'utiliser la technique de MLPA (Multiplex Ligation-dependent Probe Amplification). La MLPA est une variante de la PCR multiplexe et permet d'amplifier de multiples séquences nucléotidiques cibles avec une paire d'amorces universelles. L'amplification, de préférence par PCR, est précédée d'une étape de ligation. Chaque séquence nucléotidique à amplifier est hybridée à une paire d'oligonucléotides reconnaissant deux sites (parties ou portions) adjacents de la séquence nucléotidique ciblée. Chaque oligonucléotide de la paire comporte en outre une séquence universelle qui est complémentaire d'une des amorces. Ainsi, une fois que les paires d'oligonucléotides sont hybridées à leur séquence nucléotidique cible, elles peuvent être liguées entre elles au moyen d'une ligase. Les oligonucléotides ligués sont ensuite amplifiés par une paire d'amorces universelles.

L'étape de ligation peut éventuellement être intégrée dans le procédé de l'invention et effectuée au sein d'une unité séparée du dispositif de l'invention, par exemple dans une chambre ou un (micro)canal connecté(e) au canal 3 où a lieu l'amplification (génétique), de préférence par PCR, avec une paire d'amorces universelles.

Dans un mode de réalisation utilisant la MLPA, entre 20 séquences et 1000 séquences nucléotidiques cibles sont amplifiées et détectées dans le même échantillon.

L'échantillon est de préférence une solution aqueuse contenant l'ADN purifié ainsi que d'autres composants susceptibles d'être utilisés dans le dispositif d'amplification sous flux dynamique constant.

Ces différents composés sont de préférence introduits dans le canal 3 en même temps que le ou les échantillon(s) ou éventuellement par d'autres (micro)canaux ou réservoirs mis en contact avec le canal 3.

Ces autres composants sont des réactifs aptes à réaliser cette amplification (génétique), de préférence une amplification (génétique) par PCR, notamment une ADN polymérase, apte à obtenir une polymérisation de l'ADN dans un tampon adéquat, éventuellement supplémentée par l'addition d'autres composés (réactifs) tels que du chlorure de magnésium, des amorces, en particulier des paires d'amorces adéquates (spécifiques ou universelles) pour l'amplification (génétique), des dNTPs choisis en fonction du type d'amplification (génétique), de préférence par PCR, requise et du type de séquence(s) génétique(s) à détecter.

D'autres composants tels que des agents de blocage (saturation) de surface, de préférence de l'albumine sérique (Bovine Sérum Albumine, BSA) et du polyéthylène glycol (PEG) en tant qu'agents bloquants, ou des stabilisants, ainsi que des accélérateurs de réaction (reaction enhancers) peuvent être également ajoutés à la solution de réaction.

Le choix des séquences nucléotidiques utilisées à titre d'amorces (spécifiques ou universelles) pour l'amplification (génétique), de préférence par PCR, est fait en fonction du type de séquence(s) nucléotidique(s) à amplifier et à détecter. La sélection de ces amorces est à la portée de l'homme de l'art ainsi que leur marquage éventuel par des éléments comme des particules métalliques, de préférence des particules d'or, des colloïdes ou des éléments fluorescents.

De préférence, le marqueur est un marqueur fluorescent, de préférence constitué de cyanine (Cy5) ou d'un marqueur ayant des propriétés fluorescentes similaires.

Le marquage peut aussi être indirect, via une mise en contact des séquences amplifiées non marquées avec des secondes sondes marquées et complémentaires à une partie (ou portion) de la séquence amplifiée non hybridée aux premières sondes de capture (détection sandwich).

Les marqueurs présentant différents types de coloration peuvent être également utilisés pour le marquage de différentes séquences d'amorces de manière à obtenir une amplification des séquences d'ADN sous forme multiplexe et de manière à faciliter leur détection simultanée ou consécutive sur la tigette 4.

Chaque couleur est spécifique d'une séquence ou d'un groupe de séquences génétiques amplifiées et présentes initialement dans l'échantillon testé, et leur détection colorimétrique peut être obtenue par des moyens connus de l'homme de l'art.

Le dispositif de l'invention comporte également les éléments aptes à mesurer le signal, en particulier un signal fluorescent constitué d'un(e) ou de plusieurs points ou lignes formé(e)s par le marqueur (fluorescent) sur la tigette 4, notamment un détecteur de signal, en particulier un détecteur de signal fluorescent.

De préférence, une source de lumière génère un faisceau de lumière pour exciter la fluorescence du marqueur. La détection doit être réglée de manière à obtenir la même efficacité de détection sur la surface de la tigette 4 comprenant l'ensemble des points ou des lignes à analyser. Un détecteur utilisé dans ce contexte est une caméra CCD capable de prendre une photo de l'ensemble des points ou des lignes (ou toute autre figure, dessin, lettre ou chiffre).

En plus de l'unité de détection, le dispositif de l'invention comporte d'autres éléments aptes à faciliter cette amplification (génétique), de préférence par PCR, et cette détection, notamment une unité de chauffage (par exemple une résistance chauffante ou des blocs en aluminium) de la surface inférieure du dispositif microfluidique. Ce dispositif de chauffage étant conçu de manière à obtenir une réaction d'amplification (génétique), de préférence par PCR, et une détection par traitement thermique des parties adéquates du dispositif microfluidique.

Par exemple, les chambres 1 et 2 du dispositif de l'invention reposent sur quatre (au moins trois) blocs chauffants dont la température est contrôlée indépendamment l'une de l'autre. Trois (au moins deux) blocs servent à réguler la température de l'amplification (génétique), de préférence par PCR, et sont situés en-dessous de la chambre 1 en différentes zones du canal 3, et le quatrième (troisième) bloc est situé en-dessous de la chambre 2 au niveau de la tigette 4.

Pour que l'amplification (génétique), de préférence par PCR, s'effectue avec un haut rendement, il est important que la transition d'une zone de température à une autre soit bien définie. A cette fin, les blocs chauffants sont séparés l'un de l'autre par un espace d'environ 1 mm à environ 2 mm.

L'unité de chauffe peut également comporter un couvercle ou tout autre dispositif afin de limiter les déperditions de chaleur.

Le canal 3 comporte différentes portions portées (traitées) à différentes températures de manière à faciliter l'amplification (génétique), de préférence par PCR, tandis que la chambre de détection 2 est maintenue à une température adéquate unique.

De manière préférée, les températures peuvent varier entre environ 100 °C et environ 90°C (de préférence environ 98°C), et environ 50°C et environ 80 °C (de préférence environ 57°C) dans la zone ou chambre d'amplification 1, tandis que la zone ou chambre de détection 2 est maintenue à une température comprise entre environ 40°C et environ 60°C (de préférence environ 41°C).

L'unité de détection et l'unité de chauffage peuvent être intégrées dans un même dispositif, ou être au contraire séparées selon les besoins de l'utilisateur. Etant donné que l'amplification (génétique), de préférence par PCR, demande un temps plus long que la mesure du signal, il peut être avantageux de séparer les deux unités.

Les différentes chambres ou zones 1 et 2 sont reliées au moyen d'éléments adéquats, notamment des systèmes de tuyau (tubage) standard, de préférence en polyéthylène ou en silicone, ou via un système de pompage comportant de préférence des moyens tels que des pompes, des seringues, des valves, etc. Une pompe-seringue peut être par exemple utilisée pour assurer un pompage de l'échantillon (à température constante et contrôlée) de manière à permettre également un flux constant au travers du canal 3.

Ce système de pompe-seringue comportera de préférence un diamètre interne compris entre environ 6 mm et environ 30 mm, une vitesse de pompage comprise entre environ 0,012 mm et environ 6 mm par seconde, pour un volume de pompage minimum de l'ordre de 0,3 µl par minute.

Un flux constant à l'intérieur du dispositif peut également être assuré par une pompe péristaltique (pompe à galets) en circuit fermé, mais dont la vitesse peut être variable.

Le procédé de l'invention est réalisé d'une manière très rapide en moins de 90 minutes, de préférence en moins de 60 minutes et même en moins de 30 minutes. L'amplification (génétique), de préférence par PCR, est effectuée en moins de 60 minutes et de préférence en moins de 30 min, et la détection sur tigette en moins de 15 minutes, de préférence en moins de 5 minutes (détection et révélation comprises).

## Revendications

1. Une méthode d'amplification par PCR et de détection d'au moins une séquence nucléotidique cible éventuellement présente dans un échantillon biologique, de préférence un échantillon extrait d'un individu, ladite méthode comprenant les étapes consécutives suivantes :
a) fournir un dispositif comprenant :
- un canal (3) ayant une section comprise entre 0,01 mm et 10 mm,
- une tigette (4) en communication fluidique avec le canal (3), ladite tigette (4) comprenant soit une ou des premières sondes complémentaires et spécifiques d'au moins une première partie de séquence de la ou des séquence(s) nucléotidique(s) cible(s) amplifiée(s) ou soit au moins une première molécule d'une paire de molécules différentes et complémentaires, apte à fixer une seconde molécule différente de ladite paire, ladite seconde molécule étant fixée à la première partie de séquence de la ou des séquence(s) nucléotidique(s) cible(s) amplifiée(s);
- des moyens pour générer au moins trois zones de températures différentes et constantes, au moins deux (voir trois) zones étant situées à des emplacements différents du canal (3) et la troisième (ou quatrième) zone étant située au niveau de la tigette (4),
b) introduire dans le canal (3) une solution comprenant l'échantillon ainsi que les réactifs d'amplification de la ou des séquence(s) nucléotidique(s) cible(s),
c) générer des séquences amplifiées et éventuellement marquées de la ou des séquence(s) nucléotidique(s) cible(s) en faisant circuler la solution selon un flux dynamique constant dans le canal (3),
d) mettre en contact lesdites séquences amplifiées avec la tigette (4), et
e) générer, par hybridation entre les premières sondes et lesdites séquences amplifiées, un complexe formant un signal de détection mesurable.

2. La méthode selon la revendication 1, dans laquelle les étapes b) à e) sont générées dans le dispositif de l'étape a) conçu de manière fermée.

3. La méthode selon la revendication 1 ou 2, **caractérisée en ce que** les séquences amplifiées sont générées dans le canal (3) comprenant de multiples boucles passant sur deux ou trois zones de températures différentes, constantes et configurées pour les cycles multiples d'amplification.

4. La méthode selon l'une quelconque des revendications précédentes 1 à 4, dans laquelle la solution est introduite dans le canal (3) par une pompe-seringue permettant un déplacement de l'échantillon dans le canal (3) selon un flux dynamique constant, dans laquelle la solution est introduite et déplacée en flux dynamique constant dans le canal (3) par un dispositif d'aspiration ou dans laquelle la solution est déplacée en flux dynamique constant par une pompe péristaltique (pompe à galets).

5. La méthode selon l'une quelconque des revendications précédentes, **caractérisée en ce que** les réactifs d'amplification comportent une ou plusieurs paires d'amorces et **en ce qu'**au moins une des amorces de chaque paire est marquée, ledit marqueur de l'amorce étant choisi de préférence parmi le groupe constitué par les particules métalliques, en particulier les particules d'or, les particules colloïdales, les particules colorées ou (para)magnétiques, les particules de polystyrène ou les éléments fluorescents, de préférence le marqueur fluorescent est la cyanine (Cy5) ou un marqueur ayant des propriétés fluorescentes similaires et **en ce que** le signal de détection est de préférence constitué de une ou plusieurs lignes ou points formées par le marqueur ou **en ce que** le signal de détection est constitué de 4, 6, 8, 9, 10, 12, 14, 16 ou 32 points formés par le marqueur.

6. La méthode selon l'une quelconque des revendications précédentes, **caractérisée en ce que** l'on met en contact une partie de la séquence des séquences nucléotidiques amplifiées, non hybridée avec les premières sondes, avec une ou plusieurs secondes sondes marquées et **en ce que** la séquence nucléotidique cible est simple brin, double brin, partiellement double brin, ou une séquence d'ADNc rétro-transcrite à partir d'une séquence d'ARN.

7. La méthode selon l'une quelconque des revendications précédentes, **caractérisée en ce qu'**elle comporte préalablement à et y compris l'étape b) une réaction de MLPA (Multiplex Ligation-dependent Probe Amplification).

8. La méthode selon l'une quelconque des revendications précédentes, dans laquelle la lecture du signal est corrélée à l'identification d'une ou plusieurs infection(s) par un ou plusieurs agent(s) pathogène(s), et/ou à l'identification de la résistance d'un ou plusieurs agent(s) pathogène(s) à un ou plusieurs traitement(s) thérapeutique(s) et l'agent pathogène est de préférence une bactérie et la résistance est une de préférence une résistance à un ou plusieurs antibiotique(s) ou dans laquelle la lecture du signal est corrélée à l'identification d'une contamination d'un produit alimentaire.

9. La méthode selon l'une quelconque des revendications précédentes pour l'amplification et la détection de multiples et différentes séquences nucléotidiques présentes dans le même échantillon (conditions multiplexes).

10. La méthode selon l'une quelconque des revendications précédentes, dans laquelle la paire de molécules différentes et complémentaires est choisie parmi le groupe constitué par la streptavidine/biotine, l'avidine/biotine, la polystreptavine/biotine ou un anticorps anti-biotine/biotine.

11. Un dispositif d'amplification et de détection rapide d'au moins une séquence nucléotidique cible comprenant les moyens pour la mise en oeuvre de la méthode selon l'une quelconque des revendications précédentes et comprenant :
- un canal (3) ayant une section comprise entre 0,01 mm et 10 mm,
- une tigette (4) en communication fluidique avec le canal (3), ladite tigette (4) comprenant soit des premières sondes complémentaires et spécifiques d'une première partie de séquence des séquences nucléotidiques cibles amplifiées, ou soit au moins une première molécule d'une paire de molécules différentes et complémentaires, et apte à fixer une seconde molécule différente de ladite paire, ladite seconde molécule étant fixée à la première partie de séquence des séquences nucléotidiques cibles amplifiées,
et dans lequel le canal (3) comprend de multiples boucles passant sur les au moins deux ou trois zones de températures différentes, constantes et configurées pour les cycles multiples d'amplification (génétique) par PCR.

12. Le dispositif selon la revendication 11 comprenant des moyens pour générer au moins quatre zones de températures différentes et constantes, trois zones étant situées à des emplacements différents du canal (3) et la quatrième étant située au niveau de la tigette (4).

13. Le dispositif selon la revendication 11 ou 12, comprenant sur l'extrémité du canal (3) une pompe-seringue assurant l'introduction et le flux dynamique constant de l'échantillon au sein du canal (3).

14. Le dispositif selon l'une quelconque des revendications précédentes 11 à 13, **caractérisé en ce que** la tigette (4) comporte des premières sondes et éventuellement des secondes sondes aptes à générer un signal de détection sous forme de ligne (s) ou de point (s) sur la surface de la tigette (4) après hybridation des séquences nucléotidiques amplifiées.

15. Une trousse d'amplification par PCR et de détection d'au moins une séquence nucléotidique cible, comprenant le dispositif selon l'une quelconque des revendications précédentes 11 à 14, et les milieux et réactifs d'une amplification génétique par PCR et d'une détection oligochromatographique sur tigette (4), de préférence des amorces éventuellement marquées, éventuellement des sondes marquées et/ou éventuellement des moyens de dilution de l'échantillon, en particulier une solution tamponnée et en ce que le marqueur est choisi parmi le groupe constitué par les particules métalliques, les particules de polystyrène, les particules colorées ou (para)magnétiques, les particules colloïdales ou les éléments fluorescents, de préférence la cyanine (Cy5) ou un marqueur ayant des propriétés fluorescentes similaires et dans laquelle la paire de molécules différentes et complémentaires est choisie parmi le groupe constitué par la streptavidine/biotine, l'avidine/biotine, la polystreptavidine/biotine ou un anticorps anti-biotine/biotine.

## Patentansprüche

1. Verfahren zum Amplifizieren mittels PCR und zum Nachweis mindestens einer Zielnukleotidsequenz, die eventuell in einer biologischen Probe, vorzugsweise einer einem Individuum entnommenen Probe, vorhanden ist, wobei das Verfahren die folgenden aufeinanderfolgenden Schritte umfasst:
a) Bereitstellen einer Vorrichtung, umfassend:
- einen Kanal (3) mit einem Querschnitt zwischen 0,01 mm und 10 mm inklusive,
- einen Teststreifen oder Messstab (4) in Fluidkommunikation mit dem Kanal (3), wobei der Teststreifen oder Messstab (4) entweder eine oder erste komplementäre und spezifische Sonden mindestens eines ersten Sequenzabschnitts der amplifizierten Zielnukleotidsequenz(en) oder mindestens ein erstes Molekül eines Paares unterschiedlicher und komplementärer Moleküle umfasst, das imstande ist, ein zweites, von dem Paar unterschiedliches Molekül zu fixieren, wobei das zweite Molekül (direkt oder indirekt) am ersten Sequenzabschnitt der amplifizierten Zielnukleotidsequenz(en) fixiert ist,
- Mittel, um mindestens drei unterschiedliche und konstante Temperaturenzonen zu erzeugen, wobei sich mindestens zwei (oder drei) Zonen an unterschiedlichen Stellen des Kanals (3) befinden und sich die dritte (oder vierte) Zone im Bereich des Teststreifens oder Messstab(4) befindet,
b) Einleiten, in den Kanal (3), einer Lösung, welche die Probe sowie Amplifizierungsreagenzien der Zielnukleotidsequenz(en) umfasst,
c) Erzeugen von amplifizierten und eventuell mit der oder den Zielnukleotidsequenz(en) markierten Sequenzen, durch Zirkulieren der Lösung gemäß einem konstanten dynamischen Fluss im Kanal (3),
d) Inkontaktversetzen der amplifizierten Sequenzen mit dem Teststreifen oder Messstab(4) und
e) Erzeugen, durch Hybridation zwischen den ersten Sonden und den amplifizierten Sequenzen, eines Komplexes, welcher ein messbares Nachweissignal bildet.

2. Verfahren nach Anspruch 1, wobei die Schritte b) bis e) in der Vorrichtung von Schritt a) erzeugt werden, die geschlossen ausgebildet ist.

3. Verfahren nach Anspruch 1 oder 2, **dadurch gekennzeichnet, dass** die amplifizierten Sequenzen in dem Kanal (3) erzeugt werden, welcher viele Schleifen umfasst, die über zwei oder drei unterschiedliche, konstante, für die Mehrfach-Amplifikationszyklen konfigurierten Temperaturzonen laufen.

4. Verfahren nach einem der vorangehenden Ansprüche 1 bis 4, wobei die Lösung in den Kanal (3) von einer Pumpenspritze eingeleitet wird, die eine Verlagerung der Probe in dem Kanal (3) gemäß einem konstanten dynamischen Fluss erlaubt, wobei die Lösung von einer Ansaugvorrichtung in den Kanal (3) in konstantem dynamischen Fluss eingeleitet und verlagert wird oder wobei die Lösung in konstantem dynamischen Fluss von einer Peristaltikpumpe (Rollenpumpe) verlagert wird.

5. Verfahren nach einem der vorangehenden Ansprüche, **dadurch gekennzeichnet, dass** die Amplifizierungsreagenzien ein oder mehrere Primerpaare aufweisen und dass mindestens einer der Primer jedes Paares markiert ist, wobei der Marker des Primers vorzugsweise aus der Gruppe ausgewählt ist, die von den Metallpartikeln, vorzugsweise den Goldpartikeln, den kolloidalen Partikeln, den gefärbten oder (para)magnetischen Partikeln, den Polystyrolpartikeln oder den fluoreszierenen Elementen gebildet ist, wobei der fluoreszierende Marker das Cyanin (Cy5) ist oder ein Marker mit ähnlichen fluoreszierenden Eigenschaften, und dass das Nachweissignal vorzugsweise aus einer oder mehreren Linien oder Punkten besteht, die von dem Marker gebildet werden oder dadurch, dass das Nachweissignal aus 4, 6, 8, 9, 10, 12, 14, 16 oder 32 Punkten besteht, die von dem Marker gebildet werden.

6. Verfahren nach einem der vorangehenden Ansprüche, **dadurch gekennzeichnet, dass** ein nicht mit den ersten Sonden hybridisierter Abschnitt der Sequenz der amplifizierten Nukleotidsequenzen mit einer oder mehreren markierten zweiten Sonden in Kontakt versetzt wird und dass die Zielnukleotidsequenz einstrangig, zweistrangig, teileweise zweistrangig oder eine aus einer RNS-Sequenz reverstranskribierte cDNA-Sequenz ist.

7. Verfahren nach einem der vorangehenden Ansprüche, **dadurch gekennzeichnet, dass** es vor und in Schritt b) inbegriffen eine MLPA-Reaktion (Multiplex Ligation-dependent Probe Amplification) aufweist.

8. Verfahren nach einem der vorangehenden Ansprüche, wobei das Lesen des Signals mit der Identifizierung einer oder mehrerer Infektion(en) durch ein oder mehrere pathogene(s) Mittel und/oder mit der Identifizierung der Widerstandsfähigkeit eines oder mehrerer pathogenen(er) Mittel gegenüber einer oder mehreren therapeutischen Behandlung(en) korreliert ist und das pathogene Mittel vorzugsweise ein Bakterium ist und die Widerstandsfähigkeit vorzugsweise eine Widerstandsfähigkeit auf ein oder mehrere Antibiotikum(ka) ist oder wobei das Lesen des Signal mit der Identifizierung einer Kontamination eines Lebensmittelprodukts korreliert ist.

9. Verfahren nach einem der vorangehenden Ansprüche für das Amplifizieren und den Nachweis von multiplen und unterschiedlichen Nukleotidsequenzen, die in derselben Probe vorhanden sind (Multiplexbedingungen).

10. Verfahren nach einem der vorangehenden Ansprüche, wobei das Paar unterschiedlicher und komplementärer Moleküle aus der Gruppe ausgewählt ist, die von dem Streptavidin/Biotin, dem Avidin/Biotin, dem Polystreptavin/Biotin oder einem Anti-Biotin/Biotin-Antikörper gebildet ist.

11. Vorrichtung zum Amplifizieren und Schnellnachweis mindestens einer Zielnukleotidsequenz, umfassend die Mittel für die Umsetzung des Verfahrens nach einem der vorangehenden Ansprüche und umfassend:
- einen Kanal (3) mit einem Querschnitt zwischen 0,01 mm und 10 mm inklusive,
- einen Teststreifen oder Messstab (4) in Fluidkommunikation mit dem Kanal (3), wobei der Streifen (4) entweder eine oder erste komplementäre und spezifische Sonden mindestens eines ersten Sequenzabschnitts der amplifizierten Zielnukleotidsequenz(en) oder mindestens ein erstes Molekül eines Paares unterschiedlicher und komplementärer Moleküle umfasst, das imstande ist, ein zweites, von dem Paar unterschiedliches Molekül zu fixieren, wobei das zweite Molekül (direkt oder indirekt) an den ersten Sequenzabschnitt der amplifizierten Zielnukleotidsequenz(en) fixiert ist,
und wobei der Kanal (3) multiple Schleifen umfasst, die über die mindestens zwei oder drei verschiedenen, konstanten und für die multiplen Zyklen (genetischer) Amplifikation durch PCR konfigurierten Temperaturzonen laufen.

12. Vorrichtung nach Anspruch 11, umfassend Mittel, um mindestens vier unterschiedliche und konstante Temperaturenzonen zu erzeugen, wobei sich mindestens drei Zonen an unterschiedlichen Stellen des Kanals (3) befinden und sich die vierte Zone im Bereich des Teststreifens oder Messstab (4) befindet.

13. Vorrichtung nach Anspruch 11 oder 12, umfassend an einem Ende des Kanals (3) eine Pumpenspritze, welche das Einleiten und den dynamischen konstanten Fluss der Probe innerhalb des Kanals (3) sichert.

14. Vorrichtung nach einem der vorangehenden Ansprüche 11 bis 13, **dadurch gekennzeichnet, dass** der Teststreifen oder Messstab (4) erste Sonden und eventuell zweite Sonden aufweist, die imstande sind, ein Nachweissignal in Form einer Linie/von Linien oder eines Punkts/von Punkten auf der Oberfläche des Teststreifens oder Messstab (4) nach Hybridisierung der amplifizierten Nukleotidsequenzen zu erzeugen.

15. Kit zum Amplifizieren mittels PCR und Nachweis von mindestens einer Zielnukleotidsequenz, umfassend die Vorrichtung nach einem der vorangehenden Ansprüche 11 bis 14, und die Medien und Reagenzien einer genetischen Amplifikation mittels PCR und eines oligochromatigraphischen Nachweises auf Teststreifen oder Messstab (4), vorzugsweise eventuell markierte Primer, eventuell markierte Sonden und/oder eventuell Verdünnungsmittel der Probe, insbesondere eine gepufferte Lösung, und dass der Marker aus der Gruppe ausgewählt ist, die von den Metallpartikeln, den Polystyrolpartikeln, den gefärbten oder (para)magnetischen Partikeln, den kolloidalen Partikeln oder den fluoreszierenen Elementen gebildet ist, vorzugsweise dem Cyanin (Cy5), oder ein Marker mit ähnlichen fluoreszierenden Eigenschaften und wobei das Paar unterschiedlicher und komplementärer Moleküle aus der Gruppe ausgewählt ist, die von dem Streptavidin/Biotin, dem Avidin/Biotin, dem Polystreptavin/Biotin oder einem Anti-Biotin/Biotin-Antikörper gebildet ist.

## Claims

1. A method for amplifying by PCR and for detecting at least one target nucleotide sequence possibly present in a biological sample, preferably a sample extracted from an individual, said method comprising the following consecutive steps:
a) providing a device comprising:
- a channel (3) having a section comprised between 0.01 mm and 10 mm,
- a dipstick or test strip (4) in fluidic communication with the channel (3), said dipstick or test strip (4) comprising one or first complementary and specific probes of at least one first sequence portion of the amplified target nucleotide sequence(s) or i.e. at least one first molecule from a pair of different and complementary molecules, capable of binding a second molecule different from said pair, said second molecule being bound to the first sequence portion of the amplified target nucleotide sequence(s);
- means for generating at least three areas of different and constant temperatures, at least two (or even three) areas being located at different locations of the channel (3) and the third (or fourth) area being located at the dipstick or test strip (4),
b) introducing into the channel (3) a solution comprising the sample as well as the reagents for amplifying the target nucleotide sequence(s),
c) generating amplified sequences and possibly marked with the target nucleotide sequence(s) by having the solution circulate according to constant dynamic flow in the channel (3),
d) putting said amplified sequences in contact with the dipstick or test strip (4), and
e) generating, by hybridization between the first probes and said amplified sequences, a complex forming a measurable detection signal.

2. The method according to claim 1, wherein the steps b) to e) are generated in the device of step a) designed in a closed way.

3. The method according to claim 1 or 2, **characterized in that** the amplified sequences are generated in the channel (3) comprising multiple loops passing over two or three areas of different constant temperatures and configured for multiple amplification cycles.

4. The method according to any of the preceding claims, 1 to 4, wherein the solution is introduced into the channel (3) with a syringe pump allowing a displacement of the sample in the channel (3) according to a constant dynamic flow, wherein the solution is introduced and displaced in a constant dynamic flow in the channel (3) by a suction device or wherein the solution is displaced in a constant dynamic flow by a peristaltic pump (or roller pump).

5. The method according to any of the preceding claims, **characterized in that** the amplification reagents include one or several pairs of primers and **in that** at least one of the primers of each pair is labelled, said label of the primer being preferably selected from among the group formed by metal particles, in particular gold particles, colloidal particles, colored or (para)magnetic particles, polystyrene particles or florescent elements, preferably the fluorescent label is cyanin (Cy5) or a label having similar florescent properties and **in that** the detection signal preferably consists of one or several lines or points formed by the label or **in that** the detection signal consists of 4, 6, 8, 9, 10, 12, 14, 16 or 32 points formed by the label.

6. The method according to any of the preceding claims, **characterized in that** a portion of the sequence of amplified nucleotide sequences, non-hybridized with the first probes, is put into contact with one or several second labelled probes and **in that** the target nucleotide sequence is single-strand, double-strand, partly double-strand, or a cDNA sequence reverse transcribed from an RNA sequence.

7. The method according to any of the preceding claims, **characterized in that** it includes prior to and including step b), an MPLA (Multiple Ligation-dependent Probe Amplification) reaction.

8. The method according to any of the preceding claims, wherein the reading of the signal is correlated with the identification of one or several infection(s) by one or several pathogenic agent(s), and/or with the identification of the resistance of one or several pathogenic agent(s), to one or several therapeutic treatment(s) and the pathogenic agent is preferably a bacterium and the resistance is preferably a resistance to one or several antibiotics or wherein the reading of the signal is correlated with the identification of a contamination of a food product.

9. The method according to any of the preceding claims, for amplifying and detecting multiple and different nucleotide sequences present in the same sample (multiplex conditions).

10. The method according to any of the preceding claims, wherein the pair of different and complementary molecules is selected from among the group formed by streptavidin/biotin, avidin/biotin, polystreptavidin/biotin or an anti-biotin/biotin antibody.

11. A device for fast amplification and detection of at least one target nucleotide sequence comprising the means for applying the method according to any of the preceding claims and comprising:
- a channel (3) having a section comprised between 0.01 mm and 10 mm,
- a dipstick or test strip (4) in fluidic communication with the channel (3), said dipstick or test strip (4) comprising either first complementary and specific probes of first sequence portion of the amplified target nucleotide sequences or i.e. at least one first molecule from a pair of different and complementary molecules, and capable of binding a second molecule different from said pair, said second molecule being bound to the first sequence portion of the amplified target nucleotide sequences,
and wherein the channel (3) comprises multiple loops passing on said at least two or three areas of different, constant temperatures and configured for the multiple PCR (genetic) amplification cycles.

12. The device according to claim 11, comprising means for generating at least four areas of different, constant temperatures, three areas being located in different locations of the channel (3) and the fourth being located at the dipstick or test strip (4).

13. The device according to claim 11 or 12, comprising on the end of the channel (3) a syringe pump ensuring the introduction and the constant dynamic flow of the sample within the channel (3).

14. The device according to any of the preceding claims 11 to 13, **characterized in that** the dipstick or test strip (4) includes first probes and optionally second probes able to generate a detection signal as line(s) or point(s) on the surface of the small rod (4) after hybridization of the amplified nucleotide sequences.

15. An amplification kit by PCR and by detection of at least one target nucleotide sequence, comprising the device according to any of the preceding claims 11 to 14, and the media and reagents of genetic amplification by PCR and of a oligochromatographic detection on a dipstick or test strip (4), preferably optionally labelled primers, optionally labelled probes and/or optionally means for dilution of the sample, in particular a buffered solution and in that the label is selected from among the group formed with metal particles, polystyrene particles, colored or (para)magnetic particles, colloidal particles or fluorescent elements, preferably cyanin (Cy5) or a label having similar fluorescent properties and wherein the pair of different and complementary molecules is selected from among the group formed by streptavidin/biotin, avidin/biotin, polystreptavidin/biotin or an anti-biotin/biotin antibody.
